Europäisches Patentamt

European Patent Office

Office européen des brevets

(11) Publication number: **0 146 658**
**B1**

(12) **EUROPEAN PATENT SPECIFICATION**

(45) Date of publication of patent specification: **04.04.90**

(21) Application number: **83308026.0**

(22) Date of filing: **29.12.83**

(51) Int. Cl.⁵: **C 10 G 31/06**, C 07 C 15/02, C 07 C 7/14

(54) A method of producing and separating durene.

(43) Date of publication of application:
**03.07.85 Bulletin 85/27**

(45) Publication of the grant of the patent:
**04.04.90 Bulletin 90/14**

(84) Designated Contracting States:
**BE DE FR GB IT NL**

(56) References cited:
**US-A-2 914 582**
**US-A-2 914 586**
**US-A-3 150 197**
**US-A-3 894 102**
**US-A-4 298 695**

(73) Proprietor: **MOBIL OIL CORPORATION**
**150 East 42nd Street**
**New York New York 10017 (US)**

(72) Inventor: **Fowles, Patrick Ernest**
**3615 Indian Spring Road**
**Doylestown Pennsylvania 18901 (US)**
Inventor: **Yan, Tsoung-Yuan**
**2427 Fairmount Avenue**
**Philadelphia Pennsylvania 19130 (US)**

(74) Representative: **Cooper, John Anthony et al**
**Mobil Court 3 Clements Inn**
**London WC2A 2EB (GB)**

Courier Press, Leamington Spa, England.

## Description

This invention relates to the production and separation of durene, 1,2,4,5-tetramethylbenzene.

Of the tetraalkyl benzene isomers the most important currently is durene, 1,2,4,5-tetramethylbenzene. Durene is an important intermediate in the preparation of pyromellitic anhydride used in the manufacture of polyimide resins. These resins are used in the aerospace industry, as electrical wire coatings, and in laminate applications. Until recently durene has been produced by crystallizing it from hydrocarbon fractions such as naphtha reformate fractions boiling in the range 350°F to 425°F (177°C to 218°C). U.S. Patent Nos. 2,914,582; 2,914,586; and 3,150,197 are directed to aspects of this type of recovery.

Processes utilizing crystalline zeolites for converting synthesis gas as well as alcohols (primarily methanol) to gasoline have now been established in the art. In the conversion of synthesis gas to gasoline, synthesis gas is contacted in a first stage with a catalyst to form oxygenated compounds such as methanol and dimethyl ether. These products are then converted into hydrocarbons boiling in the gasoline range by passing them through a second catalytic bed containing a selected crystalline zeolite. U.S. Patent Nos. 3,894,102 and 4,076,761 exemplify such a process.

In two-stage processes for converting lower alcohols and ethers, such as methanol, ethanol, propanol, dimethyl ether, diethyl ether or mixtures thereof to gasoline these materials are contacted with a catalyst to produce a mixture having predominantly aliphatic or organic intermediate products. In a second stage this product with or without further modification is contacted with a catalyst such as crystalline zeolite and converted to a final product having a preponderance of hydrocarbons boiling within the range of gaseoline and distillate.

Gasoline produced by these processes ordinarily contains durene. Excessive amounts of durene in gasoline can, however, be troublesome, since durene is a solid at normal temperatures and pressrues with a melting point of 175°F (79°C), and hence it may crystallize out of the gasoline in unheated transfer lines or in storage tanks. Accordingly, it is highly desirable to remove the durene from the gasoline product or at least reduce the durene concentration.

In its broadest aspect, the present invention provides a process for separating durene from a gasoline-durene mixture, which process comprises fractionating the mixture to produce a lower boiling fraction; cooling the higher boiling fraction to crystallize durene therefrom; and separating the crystallized durene from its mother liquor characterised in that the gasoline-durene mixture is prepared by the catalytic conversion of a feed which comprises a $C_1$ to $C_3$ alcohol, ether, synthesis gas or a mixture thereof over a crystalline zeolite; and in that the higher boiling fraction is cooled to a temperature from −1 to +38°C.

More specifically, the present method comprises separating a mixture of durene and hydrocarbons boiling in the range of gaseoline in which the mixture is first fractionated into at least two fractions, one fraction, a heavy or bottoms fraction, being particularly concentrated in durene. This fraction ordinarily will be the heavier fraction having a higher boiling point. The remaining fraction or fractions will ordinarily constitute gasoline products. The heavy or bottoms fraction is cooled to a temperature at which durene begins to crystallize, whereafter the crystallized durene is separated from the mother liquor. The separated crystalline durene may then be washed with a solvent to remove residual mother liquor and the solvent returned to the process stream where it is converted to additional gasoline or other hydrocarbons.

The durene-containing feedstock may be produced from synthesis gas by contacting the synthesis gas with a catalyst thereby converting the synthesis gas to a mixture of ethers, alcohols and hydrocarbons, contacting the mixture with a second catalyst thereby converting the mixture to a stream rich in durene and hydrocarbons boiling within the range of gasoline and then further distilling the product stream to provide a bottoms product concentrated in durene and an overhead product comprising gasoline. The durene is then removed from the product as described above.

Alternatively, the feedstock may be produced by catalytic conversion of alcohols and/or ethers to a mixture rich in durene and gasoline. This mixture is then separated into gasoline and crystallized durene.

Techniques for deriving synthesis gas (a mixture of carbon monoxide and hydrogen) from coal, natural gas, naphthas, crude oil, shale oil and residua are well known. If desired the composition of the gas may be adjusted to provide a predetermined volumetric ratio of hydrogen to carbon monoxide plus carbon dioxide. The synthesis gas is then contacted with a carbon monoxide reduction catalyst in a first reaction zone to produce a reduction product containing typically of the order of 20 weight percent of ethers, alcohols, and other hydrocarbon products. The reduction product is catalytically converted by contact with a catalyst which preferably is a crystalline zeolite of the ZSM-5 type to form a major fraction of aromatics-rich high octane gasoline and a lesser fraction of useful products that include durene and a hydrogen-rich gaseous mixture that may be recycled to the fossil fuel gasifier.

Representative of the ZSM-5 type zeolites are ZSM-5, ZSM-11, ZSM-12, ZSM-23, ZSM-35 and ZSM-38. ZSM-5 is disclosed in U.S. Patent No. 3,702,886 and U.S. Patent RE 29,948; ZSM-11 is disclosed in U.S. Patent No. 3,709,979; ZSM-12 is disclosed in U.S. Patent No. 3,832,449; ZSM-23 is disclosed in U.S. Patent No. 4,076,842; ZSM-35 is disclosed in U.S. Patent No. 4,106,245 and ZSM-38 is disclosed in U.S. Patent No. 4,406,839.

The feedstock of gasoline and durene processed may also be derived from lower alcohols and/or ethers by a multi-stage process, in the first stage of which the alcohol reactant is contacted with a condensation catalyst to produce a predominantly aliphatic organic intermediate product. In a second stage the intermediate product with or without further modification is contacted with a crsytalline zeolite preferably of the ZSM-5 type to convert the intermediate product to a final product which is a hydrocarbon mixture having a preponderance of normally fluid hydrocarbons in the gasoline boiling range of up to 415°F (213°C).

Suitable lower alcohols that may be charged to the first stage of the process include methanol, ethanol, normal propanol and isopropanol and/or their corresponding ethers. The feed may consist of a relatively pure single alcohol or ether or a mixture thereof. The preferred charge is to this first stage of the process is ethanol, dimethyl ether and/or methanol. Particularly preferred charges include methanol and mixtures of methanol and dimethylether.

In the first stage of this conversion process the alcohol reactant is contacted with a condensation catalyst to produce water and a predominantly aliphatic organic intermediate product. The condensation catalyst may be any catalyst which results in the intermolecular dehydration of the alcohol reactant to form an aliphatic product of higher carbon to oxygen ratio than the feed. Solid inorganic and organic acidic catalysts such as phosphoric acid supported on kieselguhr, high surface area silica-alumina, acidic alumina, acid treated clays, bauxite and polystyrene sulfonic acids of the ion exchange type can be used.

The predominantly aliphatic intermediate, for example, dimethylether produced to the first stage of this process, without further conversion is contacted with a crystalline zeolite which preferably is selected from the group consisting of ZSM-5, ZSM-11, ZSM-12, ZSM-21 and TEA Mordenite. Of these ZSM-5 is particularly preferred. Operating conditions for this process are disclosed in U.S. Patent No. 3,928,483. As disclosed in this patent, it may also be desirable to follow the initial alcohol condensation stage by a further conversion stage. This intermediate conversion can be carried out in the effective presence of either the same catalyst as used for the first stage, or deactivated last stage catalyst.

The reaction product from the last stage of the reaction contains aliphatic and aromatic gasoline boiling-range hydrocarbons mixed with durene.

The liquid mixture of durene and gasoline, preferably containing at least 10, or more preferably at least 30 percent by weight of durene, is separated by distillation into at least two fractions to provide a lower boiling fraction or fractions that will constitute primarily the gasoline product and will be processed as may be further desired to convert it to a final gasoline product. The higher boiling point fraction has a boiling point above 330°F (166°C). It represents a fraction sufficiently concentrated in durene so that as it is further cooled or chilled durene begins to crystallize. As a result the durene crystallizes out at a relatively high temperature of about 100°F (38°C).

For efficient operation for durene recovery a temperature of 30 to 100°F (−1 to 38°C) is to be used but a temeprature of 65 to 80°F (18 to 27°C) is preferred.

The crystalline durene is then separated from the mother liquor by centrifugation, filtration or other suitable means. The separation step is preferably conducted at or only slightly above the crystallization temperature to prevent loss of durene to the solubilizing mother liquor. The mother liquor occluded on the surface of the durene can be removed by washing the separated crystals with a solvent, preferably methanol. The washing step is preferably effected at a temperature of 65°F to 80°F (18 to 27°C). Methanol is preferred as a wash solvent because it can be recycled to and, processed as part of, the feed stream. Methanol thus requires no separate recovery system as might other solvents. Other wash solvents which can be used include lower aliphatic hydrocarbons such as paraffins, olefins, alcohols, ketones, ethers, esters and cyclic hydrocarbons. Examples include propane, butane, pentane, propene and cyclopentane. Examples of solvents which, like methanol, can be recycled to the ether alcohol feed converter include ethanol, propane and dimethylether. The washed crystalline durene product can have a purity as high as 96% or higher meeting the requirements for commodity grade durene.

The quantity of wash liquid used to remove the mother liquor absorbed or occluded on the durene crystals depends to a large extent upon the degree of purity desired and the particular solvent utilized. Generally the quantity of washed liquid varies between 5 and 200 weight percent of the durene. A preferred range is between 10 and 50 weight percent. The durene crystals can be washed with solvent, in batch wise or continuous fashion with recycle of the mother liquor when advantageous. After the washing step the crystallized durene is dried and subjected to further processing as desired. For example, to decrease the volume of the durene product and consequently packaging costs, the durene can be melted before packaging.

As noted previously the source of the mixture of durene and gasoline boiling range hydrocarbons can be derived from a number of processes. Primary among these processes will be the conversion of synthesis gas to gasoline boiling range hydrocarbons and the conversion of methanol to gasoline. Either one of these processes will result in the feedstock to be processed as described previously.

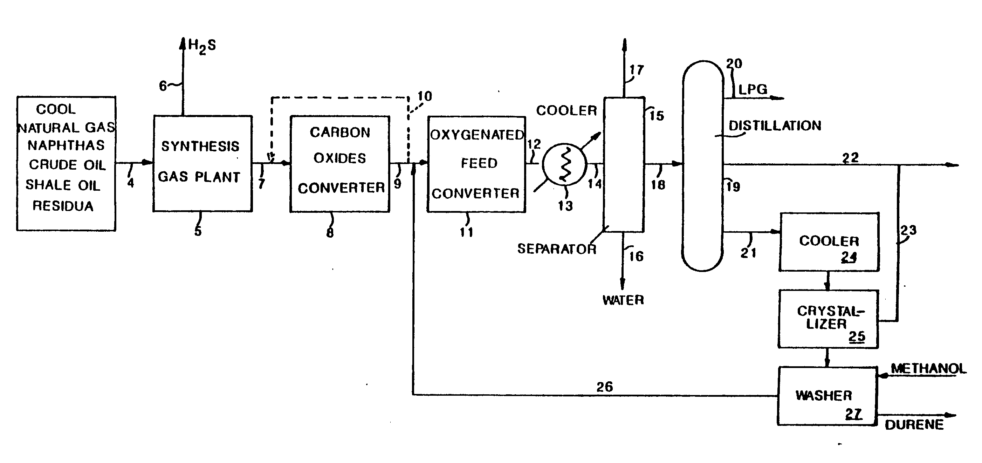

The accompanying drawing is a flow sheet of a process according to one example of the invention.

Referring to the drawing, coal, natural gas, naphthas, crude oil, shale oil, or residua, or a combination thereof, is conveyed via line 4 to a synthesis gas plant 5, where it is converted to synthesis gas. If hydrogen sulfide is produced in this plant, it may be separated and sent via line 6 to a treatment plant (not shown) for sulfur recovery. Synthesis gas, previously treated in a catalytic carbon monoxide shift converter and then reduced in carbon dioxide content by selective sorption, is conveyed via line 7 to a first reaction zone 8, where it is at least partially catalytically converted to produce a carbon monoxide reduction product that contains at least 20% by weight of alcohols, ethers and other hydrocarbon products. Part or all of the unconverted synthesis gas may be separated from such reduction product and recycled via line 10, but it is preferred to convey the total mixture via line 9 to a second reaction zone 11 where catalytic conversion to hydrocarbons and steam occurs. The reaction products from the second reaction zone 11 are conveyed via line 12 to a cooler, 13, and the cooled products are then conveyed via line 14 to a separator 15 (the cooler 13, line 14 and separator 15 may be one integral unit). Water is removed from separator 15 via line 16, gases via line 17, and liquid hydrocarbon products via line 18. The liquid hydrocarbon products are conveyed via line 18 to a distillation tower 19. In distillation tower 19 the liquid hydrocarbon products are fractionated into an overhead product of propane and butanes (LPG) which is removed via line 20, an intermediate gasoline cut which is removed via line 22 and a heavier gasoline cut, which is rich in durene and is removed via line 21. This heavier-durene cut has a boiling point above 330°F to 380°F (165°C to 193°C). The actual temperature at which this cut is made will depend in part on how much durene it is desired to recover. It is this cut which is processed further to separate the durene therefrom.

The heavier gasoline cut is then diverted by way of line 21 through a heat exchanger (not shown) and thence into a crystallization system (cooler 24 and crystallizer 25) wherein the gasoline-durene mixture is cooled to a temperature sufficient to induce the durene present to crystallize, thereby forming a mixture of durene crystals and mother liquor. With the process described the stream is surprisingly rich in durene and low in durene isomers which are difficult to separate.

The durene crystals are then separated from the mother liquor, which is returned by way of line 23 to line 22 carrying the intermediate gasoline cut. The separated durene crystals are then washed with methanol in a washer 27, removed from the washer and then dried. The effluent methanol wash liquid is passed back into the methanol conversion system by way of line 26.

The intermediate gasoline cut leaving the distillation tower 19 by way of line 22 is subjected to further processing, such as hydrotreating, to yield a desired gasoline product. In some cases, however, it may be preferred to omit line 22 and pass the entire gasoline cut by way of the line 21 through the durene crystallization system 24, 25, 27.

Having described the invention, the following example is given to illustrate the invention without limiting the scope thereof. Parts and percentages are by weight unless expressly stated otherwise and the reference numerals designate parts of the flow sheet previously discussed.

Example

A feed stream of ethers, alcohols and other hydrocarbons is introduced into converter 11 and converted to an aromatic stream containing approximately 8% of durene. The stream is passed through cooler 13 and into separator 15 where the liquid aromatic product is recovered and passed into the distillation tower 19. In distillation tower 19 the aromatic product obtained is fractionated into an overhead stream, a light gasoline stream and a heavier gasoline stream. The distillation column operates at a temperature of between 340 and 350°F (171 and 177°C) and a pressure of 0 psig (101 kPa). The gasoline product emerging from the distillation column 19 contains between 40 and 60% of durene. It is desired in this case to remove approximately 50% of the durene from the gasoline product to meet the durene concentration limitation of the gasoline pool. All the gasoline product is removed through line 21 and passed through the crystallization system. The product stream is passed first through a crystallizer 25 operating at a temperature of 70°F (21°C) where the crystallized durene is recovered and washed at 27 with methanol at a temperature of about 70°F (21°C). The crystallized durene is then removed and dried yielding a product of 97% purity.

**Claims**

1. A process for separating durene from a gasoline-durene mixture, which process comprises fractionating the mixture to produce a lower boiling fraction; cooling the higher boiling fraction to crystallize durene therefrom; and separating the crystallized durene from its mother liquor characterised in that the gasoline-durene mixture is prepared by the catalytic conversion of a feed with comprises a $C_1$ to $C_3$ alcohol, ehter, synthesis gas or a mixture thereof over a crystalline zeolite; and in that the higher boiling fraction is cooled to a temperature from $-1$ to $+38°C$.

2. A process according to claim 1 wherein crystallized durene is washed with a solvent to produce a wash liquid which is thereafter converted catalytically to durene and liquid hydrocarbons which become part of the product stream subjected to fractionation.

3. A process according to claim 1 or 2 wherein crystallized durene is washed with a solvent to produce a wash liquid which is thereafter added to the feed to the reaction zone.

4. A process according to claim 1 or 2 wherein the solvent is methanol.

5. A process according to any preceding claim wherein the durene is crystallized at 18 to 27°C (65 to 80°F).

6. A process according to any preceding claim wherein the higher boiling fraction contains at least 30% by weight of durene.

## Patentansprüche

1. Verfahren zur Abtrennung von Durol aus einer Benzin/Durol-Mischung, wobei dieses Verfahren umfaßt: Fraktionierung der Mischung, um eine geringer siedende Fraktion zu erzeugen, Abkühlung der höher siedenden Fraktion, um Durol daraus auszukristallisieren, und Abtrennung des auskristallisierten Durols aus der Mutterflüssigkeit, dadurch gekennzeichnet, daß die Benzin/Durol-Mischung durch katalytische Umwandlung einer Zufuhr, die $C_1$-$C_3$-Alkohol, Ether, Synthesegas oder eine Mischung davon umfaßt, über einem kristallinen Zeolith hergestellt wird, und daß die höher siedende Fraktion auf eine Temperatur von −1 bis +38°C abgekühlt wird.

2. Verfahren nach Anspruch 1, worin das kristallisierte Durol mit einem Lösungsmittel gewaschen wird, um eine Waschflüssigkeit zu erzeugen, die danach katalytisch in Durol und flüssige Kohlenwasserstoffe umgewandelt wird, die ein Teil des Produktstromes werden, der der Fraktionierung unterzogen wird.

3. Verfahren nach Anspruch 1 oder 2, worin das kristallisierte Durol mit einem Lösungsmittel gewaschen wird, um eine Waschlüssigkeit zu erzeugen, die danach der Zuführ zur Reaktionszone zugegeben wird.

4. Verfahren nach Anspruch 1 oder 2, worin das Lösungsmittel Methanol ist.

5. Verfahren nach einem der vorstehenden Ansprüche, worin das Durol bei 18 bis 27°C (65 bis 80°F) kristallisiert wird.

6. Verfahren nach einem der vorstehenden Ansprüche, worin die höher siedende Fraktion mindestens 30 Gew.-% Durol enthält.

## Revendications

1. Un procédé pour séparer du durène d'un mélange d'essence et de durène comprenant le fractionnement du mélange pour produire une fraction ayant une gamme d'ébullition inférieure; le refroidissement de la fraction agent une gamme d'ébullition supérieure pour faire cristalliser le durène à partir de celle-ci; et la séparation du durène cristallisé de sa liqueur-mère, caractérisé en ce que le mélange essence-durène est préparé par la conversion catalytique d'une charge d'alimentation qui comprend un alcool, un éther en $C_{1-3}$, un gaz de synthése ou leur mélange sur une zéolite cristalline; et que la fraction ayant une gamme d'ébullition supérieure est refroidie à une température de −1°C à +38°C.

2. Un procédé suivant la revendication 1, caractérisé en ce que le durène cristallisé est lavé avec un solvant pour produire un liquide de lavage qui est ensuite converti catalytiquement en durène et hydrocarbures liquides qui deviennent une partie du courant de produit soumis au fractionnement.

3. Un procédé suivant la revendication 1 ou la revendication 2, caractérisé en ce que la durène cristallisé est lavé avec un solvant pour produire un liquide de lavage qui est ensuite ajouté à la charge envoyée dans la zone de réaction.

4. Un procédé suivant la revendication 1 ou la revendication 2, caractérisé en ce que le solvant est le méthanol.

5. Un procédé suivant l'une quelconque des revendications précédentes, caractérisé en ce que le durène est cristallisé entre 18 et 27°C (65 et 80°F).

6. Un procédé suivant l'une quelconque des revendications précédentes, caractérisé en ce que la fraction ayant une gamme d'ébullition supérieure contient au moins 30% en poids de durène.

COOL
NATURAL GAS
NAPHTHAS
CRUDE OIL
SHALE OIL
RESIDUA

SYNTHESIS GAS PLANT 5

H₂S 6

CARBON OXIDES CONVERTER 8

OXYGENATED FEED CONVERTER 11

COOLER 15

SEPARATOR

WATER 16

DISTILLATION

LPG 20

COOLER 24

CRYSTAL-LIZER 25

WASHER 27

METHANOL

DURENE